Europäisches Patentamt

European Patent Office

Office européen des brevets

(11) Veröffentlichungsnummer: **0 028 712**
**B1**

# (12) EUROPÄISCHE PATENTSCHRIFT

(45) Veröffentlichungstag der Patentschrift:
**18.05.83**

(51) Int. Cl.³: **A 61 B 17/16, A 61 F 1/03**

(21) Anmeldenummer: **80106149.0**

(22) Anmeldetag: **10.10.80**

(54) **Vorrichtung zum Entfernen einer Knochenzementröhre bei einer Reimplantation eines künstlichen Oberschenkelhalskopfes.**

(30) Priorität: **06.11.79 DE 2944710**

(43) Veröffentlichungstag der Anmeldung:
**20.05.81 Patentblatt 81/20**

(45) Bekanntmachung des Hinweises auf die Patenterteilung:
**18.05.83 Patentblatt 83/20**

(84) Benannte Vertragsstaaten:
**AT BE CH DE FR GB IT LI NL SE**

(56) Entgegenhaltungen:

**CH-A-566 127**
**DE-B-2 101 002**
**DE-C-183 745**
**DE-C-741 650**
**FR-A-1 390 415**
**US-A-2 641 831**

(73) Patentinhaber: **Dimakos, Christos, Dr.,**
**Prinzenstrasse 11, D-4600 Dortmund 1 (DE)**
Patentinhaber: **Radtke, Gerhard, Neue Sendstrasse 2b,**
**D-4600 Dortmund 12 (DE)**

(72) Erfinder: **Dimakos, Christos, Dr., Prinzenstrasse 11,**
**D-4600 Dortmund 1 (DE)**
Erfinder: **Radtke, Gerhard, Neue Sendstrasse 2b,**
**D-4600 Dortmund 12 (DE)**

(74) Vertreter: **Patentanwälte Meinke und Dabringhaus**
**Dipl.-Ing. J. Meinke Dipl.-Ing. W. Dabringhaus,**
**Westenhellweg 67, D-4600 Dortmund 1 (DE)**

## Vorrichtung zum Entfernen einer Knochenzementröhre bei einer Reimplantation eines künstlichen Oberschenkelhalskopfes

Die Erfindung richtet sich auf eine Vorrichtung zum Entfernen einer Knochenzementröhre, insbesondere bei einer Reimplantation eines künstlichen Oberschenkelhalskopfes, aus einem Knochenhohlraum, mit einem an der Knochenzementröhre festlegbaren Extraktor, der als Hohlrohr ausgebildet ist, in dessen Innerem ein Betätigungselement für ein unterhalb des Hohlrohres, dazu konzentrisch angeordnetes, in der Gebrauchslage sich am unteren, stirnseitigen Ende der Knochenzementröhre anlegendes Spreizelement geführt ist, wobei das Spreizelement mit die Spreizung bewirkenden, abgeschrägten Aussenflächen und einem Innengewinde ausgestattet ist, wobei ferner das Betätigungselement mit einem Gewindebereich, der in der Gebrauchslage mit dem Innengewinde im Spreizelement zusammenwirkt, und am gegenüberliegenden Endbereich mit einem Handgriff und mit einer Anschlagschulter zum Anlegen am Hohlrohr ausgerüstet ist, wobei ferner am Hohlrohr im Bereich des in der Gebrauchslage freien Endes ein Anschlag und ein auf dem Hohlrohr axial verschiebbarer, gegen den Anschlag führbarer achssymmetrischer Schlagkörper vorgesehen sind.

Bei einer Allo-Plastik am Hüftgelenk ist es bekannt, einen Fortsatz am künstlichen Schenkelhalskopf in einer Höhlung im Oberschenkelknochen mit Hilfe von Knochenzement festzulegen.

Derartige Implantate können nicht beliebig lange im Oberschenkelknochen verbleiben, da es zu Lockerungserscheinungen insbesondere im Grenzschichtbereich zwischen Knochenzement und Fermur kommt. Muss ein solches Implantat ausgewechselt werden, so verbleibt nach dessen Entfernung eine praktisch zylindrische Knochenzementröhre im Knochen, die zur Reimplantation vollständig entfernt werden muss.

Das Entfernen des alten Knochenzementes geschah bisher durch Ausbohren von oben bzw. durch Bypasslegung schräg von unten, um alle Knochenzementteile lösen zu können und mittels Zangen bzw. sonstiger mechanischer Mittel oder auch mittels Absaugen gänzlich aus der Höhlung im Oberschenkelknochen zu entfernen. Es ist auch bekannt, sich zu diesem Zweck eines Extraktors zu bedienen, der an seinem Kopf ein Pfropfengewinde aufweist, welches möglichst weit unten in die Knochenzementröhre eingeschnitten wird, um auf diese Art und Weise genügend Halt zum Austreiben der Knochenzementröhre zu erhalten. Dieses bekannte Instrument hat u.a. den erheblichen Nachteil, dass insbesondere bei älteren zu operierenden Personen die Gefahr besteht, dass die Knochenwandung aufsplittert, da die durch das Pfropfengewinde erzeugte Radialspannung grösser sein kann als die Festigkeit der Knochen. Auch ist mit dem bekannten Instrument nicht sichergestellt, dass im Fusspunkt der im Knochen befindlichen Höhlung sämtlicher Knochenzement abgelöst wird.

Der Erfindung liegt die Aufgabe zugrunde, beim Austreiben einer Knochenzementröhre Radialkräfte auf den Knochen zu vermeiden und gleichzeitig ein vollständiges Abtragen der Knochenzementröhre sicherzustellen, wobei die Knochenzementröhre von der Seite aus entfernt werden muss, die zur Entfernung des Implantats operativ ohnehin eröffnet werden musste.

Mit einer Vorrichtung der eingangs bezeichneten Art wird diese Aufgabe gemäss der Erfindung dadurch gelöst, dass das Spreizelement ein Hohlkegelstumpf ist, dessen grösserer Aussendurchmesser in der Einführlage der Vorrichtung etwa dem Aussendurchmesser des Hohlrohres entspricht und in dessen Kegelmantel, ausgehend vom Bereich des grösseren Durchmessers, Längsschlitze zur Erzeugung von Spreizflügeln eingebracht sind, und dass das Hohlrohr an seinem an das Spreizelement angrenzenden Ende mit einem mit Aussengewinde versehenen Bereich zur Festlegung des Hohlrohres in einem in der Knochenzementröhre anzubringenden Innengewinde versehen ist und das dem Spreizelement zugewandte Hohlrohrende als umlaufende Kegelfläche ausgebildet ist, deren Aussendurchmesser in Richtung auf das Hohlrohrende hin abnimmt.

Aus der US-A-2 641 831 ist eine Vorrichtung bekannt zum Entfernen einer Röhre aus einer vorhandenen Höhlung, mit einem an der Röhre festlegbaren Extraktor, wobei der Extraktor als Hohlrohr ausgebildet ist, in dessen Innerem ein Betätigungselement für ein unterhalb des Hohlrohres, dazu konzentrisch angeordnetes, in der Gebrauchslage sich am unteren, stirnseitigen Ende der Röhre anlegendes Spreizelement geführt ist, und wobei das Spreizelement als hohles Keilelement mit Innengewinde ausgebildet ist. Diese bekannte Vorrichtung ist nicht für den medizinischen Einsatz vorgesehen und auch hierfür nicht geeignet, da dort das auszutreibende Hohlrohr beidseitig eingespannt wird, womit axiale Kräfte auf dieses in erheblichem Masse ausgeübt werden, die beim Austreiben einer Knochenzementröhre zu deren vorzeitiger Zerstörung führen würden, was die Erfindung zu vermeiden sucht. Bei den bekannten Vorrichtungen werden auch nur kleine Bereiche des unteren Endes des auszutreibenden Hohlrohres beim Austreiben belastet, was wiederum bei einer Knochenzementröhre zu deren Zerstörung führen müsste. Im Gegensatz hierzu muss bei dem operativen Einsatz der Erfindung für eine möglichst grossflächige Anlage an der unteren Stirnseite der Knochenzementröhre gesorgt sein.

Aus der FR-A-1 390 415 ist ein hydraulisch betreibbares Extraktorelement für Haltebuchsen aus Metall bekannt, bei dem freie hakenförmige Federlaschen zunächst durch die Innenbohrung der auszutreibenden Buchse geführt werden und dann über Schrägflächen an einem Innendorn beim Herausziehen nach aussen über eine Kolben-/Zylindereinheit ausgetrieben werden, um die freie Stirnseite der Buchse an der Unterseite zu erfassen. Diese bekannte Vorrichtung eignet sich überhaupt nicht zum Einsatz im medizinischen Bereich, da sie die Austreibkräfte direkt auf die Umgebung der Buchse, z.B. einer Maschinenwand, überträgt, was im medizinischen Bereich unmöglich ist.

Mit der erfindungsgemässen Vorrichtung ist es möglich, nach Einbringen des Extraktors in die Höhlung mit dem Spreizelement die Knochenzementröhre über einen sehr grossen Bereich zu untergreifen und dann auszutreiben.

In Ausgestaltung sieht die Erfindung vor, dass die Längsschlitze des Spreizelementes etwa bis zur Mitte der Längserstreckung des Hohlkegelstumpfes reichen, wodurch sich eine besonders gute Betätigungsmöglichkeit der Spreizflügel ergibt.

Um beim Betätigen des das Spreizelement beaufschlagenden Elementes ein Durchdrehen des Spreizelementes zu verhindern, ist nach der Erfindung in weiterer Ausgestaltung auch vorgesehen, dass die Kegelfläche mit reibungserhöhenden Nuten, Nocken oder Einfräsungen ausgerüstet ist.

Nach der Erfindung kann auch vorgesehen sein, dass der Anschlag für den Schlagkörper und ein am Hohlrohr vorgesehener Handgriff jeweils einstückig ausgebildet sind.

Die Erfindung ist nachstehend anhand der Zeichnung beispielsweise näher erläutert. Diese zeigt in

Fig. 1 eine Gesamtansicht der Vorrichtung in perspektivischer Darstellung,

Fig. 2 den Arbeitsbereich der Vorrichtung in Einführstellung in vergrössertem Massstab und

Fig. 3 den Arbeitsbereich der Vorrichtung nach Spreizen des Spreizelementes nach Fig. 2.

Die in den Figuren allgemein mit 1 bezeichnete Vorrichtung wird im wesentlichen von einem als Hohlrohr 2 ausgebildeten Extraktor gebildet, dessen in der Gebrauchslage im Inneren des mit 3 bezeichneten Oberschenkelknochens angeordnetes Ende 4 mit einem Gewinde 5 versehen, während am gegenüberliegenden Ende 6 ein mit einer Handhabe 7 versehenes Anschlagstück 8 fest verbunden ist. Auf dem Extraktor 2 ist ein hohlzylinderförmiger Schlagkörper 9 in Richtung des Doppelpfeiles 10 verschiebbar angeordnet.

Im Inneren des Extraktors 2 ist ein Betätigungselement 11 angeordnet, welches sich mit einer Anschlagschulter 12 auf der dem Schlagkörper 9 abgewandten Seite des Anschlages 8 an diesem abstützt. Das Betätigungselement ist ebenfalls mit einer Handhabe 13 ausgerüstet, mit der das Betätigungselement insbesondere in Richtung des Doppelpfeiles 14 drehbar ist.

Das der Handhabe 13 gegenüberliegende Ende 15 des Betätigungselementes 11 ist mit einem Gewindebereich 16 versehen, auf dem ein Spreizelement 17 mit einem Innengewinde 18 angeordnet ist.

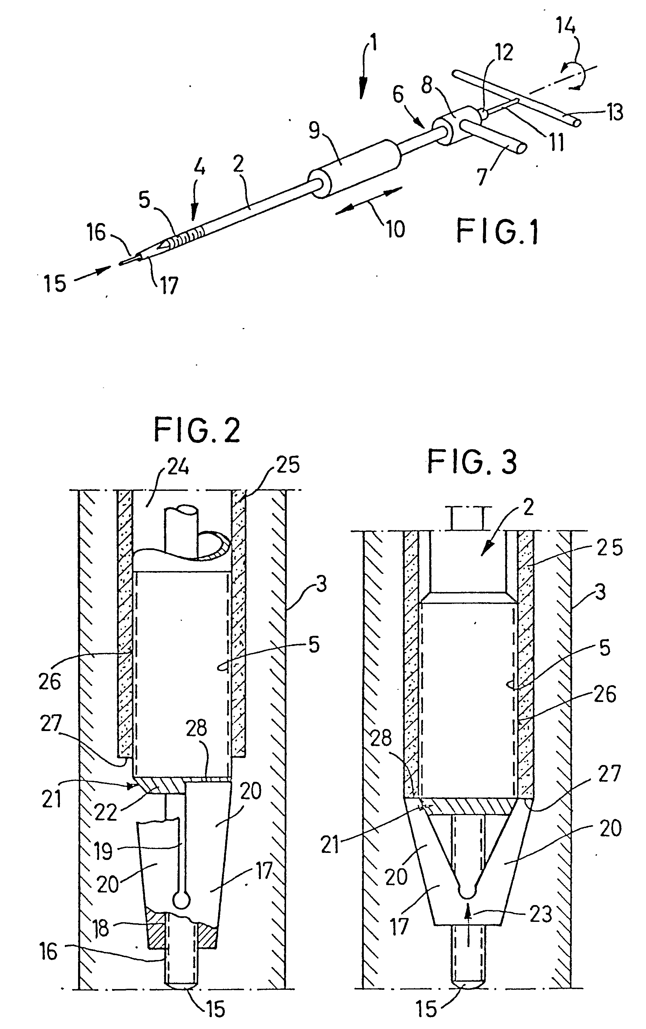

Das Spreizelement 17 ist, wie insbesondere aus den Fig. 2 und 3 hervorgeht, kegelstumpfförmig ausgebildet, wobei in der in Fig. 2 dargestellten Einführlage der dem Extraktor 2 zugewandte grössere Durchmesser des Kegelstumpfes etwa dem Aussendurchmesser des Extraktors entspricht. Von dieser Seite her sind in das Spreizelement Schlitze 19 eingebracht, die auf diese Art und Weise z.B. vier Spreizflügel 20 bilden.

Das untere freie Ende 4 des Extraktors ist mit einer Kegelfläche 21 ausgerüstet, die mit reibungserhöhenden Stegen 22 versehen ist, die in der Gebrauchslage beim Verdrehen der Handhabe 13 das Mitdrehen des Spreizelementes 17 verhindern, so dass dieses in Richtung des Pfeiles 23 (Fig. 3) über das untere Ende 4 des Extraktors 2 gezogen werden kann und sich dabei aufweitet.

Die Wirkungsweise der Vorrichtung ist die folgende:

Nachdem ein Implantat aus einem Oberschenkelknochen 3 entfernt worden ist, bleibt eine sich leicht nach oben erweiternde Höhlung 24 (Fig. 2) erhalten, die mit einer Knochenzementröhre 25 ausgekleidet ist. Mit einem Bohrer wird der ursprünglich geschlossene Fussbereich der Knochenzementröhre 25 aufgebohrt und anschliessend das untere Ende der Knochenzementröhre 25 mit einem Innengewinde 26 versehen. Durch den konischen Verlauf der Höhlung 24 ist nur das untere Ende der Höhlung bei entsprechender Bemassung des Gewindeschneiders mit einem Gewinde ausgerüstet.

Nunmehr wird die Vorrichtung von oben in die Sackbohrung eingeführt und das Extraktorrohr 2 mit seinem Gewinde 5 in das Gewinde 26 eingeschraubt, wobei das Spreizelement 17 bis unter das untere Ende 27 der Knochenzementröhre eingeführt wird. Durch Festhalten des Extraktors 2 mit der Handhabe 7 und Drehen des Betätigungselementes 11 mit der Handhabe 13 in einer der Richtungen des Doppelpfeiles 14 wird das Spreizelement an seiner Drehung durch die Stege 22 gehindert, in Richtung des Pfeiles 23 über die Gewindeverbindung 16/18 nach oben gezogen und weitet sich so weit aus, dass die Oberkanten 28 der Spreizflügel 20 die unteren Enden 27 der Knochenzementröhre 25 in der in Fig. 3 wiedergegebenen Weise untergreifen. Das Mass des Aufspreizens kann z.B. über einen Monitor und Dauerröntgenüberwachung vom behandelnden Chirurgen bestimmt werden.

Nunmehr wird der Schlagkörper 9 betätigt, der aufgrund seiner konzentrischen Anordnung eine genau mittige Krafteinleitung ermöglicht. Durch die ruckartigen Schläge wird in der Regel die Knochenzementröhre als Ganzes ausgeschlagen, ohne dass Rückstände in der Höhlung zurückbleiben, was auch dadurch begünstigt wird, dass sich die Öffnung leicht konisch nach oben erweitert.

Natürlich ist das beschriebene Ausführungsbeispiel noch in vielfacher Hinsicht abzuändern, ohne den Grundgedanken der Erfindung zu verlassen. So ist die Erfindung insbesondere nicht auf die in den Zeichnungen dargestellte spezielle Gestaltung des kegelstumpfförmigen Spreizelementes, der Handhaben oder des Schlagkörpers beschränkt. In gleicher Weise können auch über das Betätigungselement zu steuernde, schwenkbar am unteren Ende des Extraktors angeordnete Nocken ausgespreizt werden o. dgl. mehr. Auch kann nach der Erfindung der gewindelose Teil des Extraktors einen geringeren Durchmesser aufweisen, als der mit Gewinde versehene vordere Bereich, was insbesondere dann zweckmässig ist, wenn die Höhlung sich nicht nach aussen erweitert, sondern durchgehend den gleichen Durchmesser aufweist.

**Patentansprüche**

1. Vorrichtung (1) zum Entfernen einer Knochenzementröhre (25), insbesondere bei einer Reimplantation eines künstlichen Oberschenkelhalskopfes, aus einem Knochenhohlraum (24), mit einem an der Knochenzementröhre (25) festlegbaren Extraktor (2), der als Hohlrohr (2) ausgebildet ist, in dessen Innerem ein Betätigungselement (11) für ein unterhalb des Hohlrohres (2), dazu konzentrisch angeordnetes, in der Gebrauchslage sich am unteren, stirnseitigen Ende der Knochenzementröhre (25) anlegendes Spreizelement (17) geführt ist, wobei das Spreizelement mit die Spreizung bewirkenden, abgeschrägten Aussenflächen und einem Innengewinde ausgestattet ist, wobei ferner das Betätigungselement (11) mit einem Gewindebereich (16), der in der Gebrauchslage mit dem Innengewinde (18) im Spreizelement (17) zusammenwirkt, und am gegenüberliegenden Endbereich mit einem Handgriff und mit einer Anschlagschulter (12) zum Anlegen am Hohlrohr (2) ausgerüstet ist, wobei ferner am Hohlrohr (2) im Bereich des in der Gebrauchslage freien Endes (6) ein Anschlag (8) und ein auf dem Hohlrohr (2) axial verschiebbarer, gegen den Anschlag (8) führbarer achssymmetrischer Schlagkörper (9) vorgesehen sind, dadurch gekennzeichnet, dass das Spreizelement (17) ein Hohlkegelstumpf ist, dessen grösserer Aussendurchmesser in der Einführlage der Vorrichtung (1) etwa dem Aussendurchmesser des Hohlrohres (2) entspricht und in dessen Kegelmantel, ausgehend vom Bereich des grösseren Durchmessers, Längsschlitze (19) zur Erzeugung von Spreizflügeln (20) eingebracht sind, und dass das Hohlrohr (2) an seinem an das Spreizelement (17) angrenzenden Ende (4) mit einem mit Aussengewinde (5) versehenen Bereich zur Festlegung des Hohlrohres (2) in einem in der Knochenzementröhre (25) anzubringenden Innengewinde (26) versehen ist und das dem Spreizelement (17) zugewandte Hohlrohrende (4) als umlaufende Kegelfläche (21) ausgebildet ist, deren Aussendurchmesser in Richtung auf das Hohlrohrende (4) hin abnimmt.

2. Vorrichtung nach Anspruch 1, dadurch gekennzeichnet, dass die Längsschlitze (19) etwa bis zur Mitte der Längserstreckung des Hohlkegelstumpfes reichen.

3. Vorrichtung nach Anspruch 1, dadurch gekennzeichnet, dass die Kegelfläche (21) mit reibungserhöhenden Nuten, Nocken oder Einfräsungen (22) ausgerüstet ist.

4. Vorrichtung nach Anspruch 1, dadurch gekennzeichnet, dass der Anschlag (8) für den Schlagkörper (9) und ein am Hohlrohr (2) vorgesehener Handgriff (7) jeweils einstückig ausgebildet sind.

**Revendications**

1. Dispositif (1) destiné à extraire un tube en ciment (25) d'une cavité osseuse (24), en vue notamment d'une réimplantation de tête fémorale artificielle, ce dispositif étant du type comportant un extracteur (2) en forme de conduit creux susceptible d'être fixé sur le tube en ciment (25), dans l'intérieur duquel est guidé un organe de manoeuvre (11) pour un élément d'écartement (17) disposé au-dessous du conduit creux (2) de façon concentrique à ce dernier, et qui dans la position d'utilisation prend appui sur la face frontale inférieure du tube en ciment (25), l'élément d'écartement étant muni d'un filetage intérieur et de faces extérieures biseautées provoquant son écartement, alors qu'en outre l'organe de manoeuvre (11) comporte une partie filetée (16) qui coopère dans la position d'utilisation avec le filetage intérieur (18) de l'élément d'écartement (17), et comprend à l'extrémité opposée une poignée et un épaulement (12) de butée venant en appui sur le conduit creux (2), alors que le conduit creux comprend en outre une butée (8) dans la zone de son extrémité libre (6) en position de travail et une masse à percussion (9) mobile axialement sur le conduit creux (2) et déplaçable symétriquement à l'axe de la butée (8), caractérisé en ce que l'élément d'écartement (17) est un cône tronqué creux (2) dont le grand diamètre extérieur correspond approximativement au diamètre extérieur du conduit creux dans la position d'introduction du dispositif, des fentes longitudinales (19) pour la création d'ailettes (20) d'écartement étant ménagées dans l'enveloppe conique à partir de la zone du grand diamètre, que le conduit creux (2) comprend, à son extrémité (4) adjacente à l'élément d'écartement (17), une partie filetée (5) extérieurement pour la fixation du conduit creux (2) dans un filetage intérieur (26) aménagé dans le tube en ciment (25), et que l'extrémité (4) du conduit creux orientée vers l'élément d'écartement (17) est conçue en forme de face conique enveloppante (21) dont le diamètre extérieur diminue en direction de l'extrémité (4) du conduit creux (2).

2. Dispositif selon la revendication 1, caractérisé en ce que les fentes longitudinales (19) s'étendent approximativement jusqu'au milieu de l'extension longitudinale du cône tronqué creux.

3. Dispositif selon la revendication 1, caractérisé en ce que la face conique (21) comporte des rainures, cames ou alésages (22) accroissant le frottement.

4. Dispositif selon la revendication 1, caractérisé en ce que la butée (8) pour la masse percutante (9) et une poignée (7) prévue sur le conduit creux (2) sont exécutées d'une seule pièce.

**Claims**

1. Apparatus (1) for removing a tubular portion of bone cement (25), in particular upon re-implantation of an artificial thigh neck head portion, from a bone cavity (24), comprising an extractor (2) which can be fixed to the bone cement tubular portion (25) and which is in the form of a hollow tube (2), in the interior of which is guided an actuating element (11) for a spreader element (17) which is disposed below the hollow tube (2) concentrically thereto and which in the position of use bears against the lower front end of the bone cement tubular portion (25), wherein the spreader element is provided with inclined external surfaces for producing the spreading action, and an internal thread, wherein in addition the actuating element (11) is provided with a threaded portion (16) which in the position of use co-operates with the internal thread (18) in the spreader element (17), and wherein the actuating element (11) is provided at the opposite end region with a handle and with an abutment shoulder (12) for bearing against the hollow tube (2), wherein moreover provided on the hollow tube (2) in the region of the end (6) which is the free end in the position of use is an abutment (8) and an axially symmetrical impact member (9) which is axially slidable on the hollow tube (2) and which can be driven against the abutment (8), characterised in that the spreader element (17) is a hollow truncated cone, the larger outside diameter of which, in the insertion position of the apparatus (1), substantially corresponds to the outside diameter of the hollow tube (2), while disposed in the tapered peripheral portion of the spreader element, extending from the larger-diameter region thereof, are longitudinal slots (19) for forming spreader wing portions (20), and that, at its end (4) adjoining the spreader element (17), the hollow tube (2) is provided with a region having an external thread (5) for fixing the hollow tube (2) in an internal thread (26) to be formed in the bone cement tubular portion (25), and that the end (4) of the hollow tube (2), which is towards the spreader element (17), is in the form of a peripherally extending tapered surface (21), the outside diameter of which decreases towards the end (4) of the hollow tube.

2. Apparatus according to claim 1 characterised in that the longitudinal slots (19) extend substantially to the middle of the longitudinal dimension of the hollow truncated cone.

3. Apparatus according to claim 1 characterised in that the tapered surface (21) is provided with friction-increasing grooves, projections or milled recesses (22).

4. Apparatus according to claim 1 characterised in that the abutment (8) for the impact member (9) and a handle (7) provided on the hollow tube (2) are respectively integral.

FIG.1

FIG.2

FIG.3